# EUROPÄISCHE PATENTSCHRIFT

(11) **EP 0 469 494 B1**
(45) Veröffentlichungstag und Bekanntmachung des Hinweises auf die Patenterteilung: **25.01.1995**
(21) Anmeldenummer: 91112667.0
(22) Anmeldetag: 27.07.1991
(51) Int. Cl.: C12N 11/08, C12N 11/14, C12P 19/24

(54) **Enzymträger auf anorganischer Basis und trägergebundene Enzyme**
Inorganic based enzyme carrier and immobilized enzymes
Support pour enzymes à base inorganique et enzymes immobilisées

(30) Priorität: 02.08.1990 DE 4024491
(43) Veröffentlichungstag der Anmeldung: 05.02.1992
(73) Patentinhaber: Kali-Chemie Aktiengesellschaft, D-30173 Hannover (DE)
(72) Erfinder: Bonse, Dirk, W-3160 Lehrte-Arpke (DE); Schindler, Hubert, W-3162 Uetze-Dollbergen (DE); Müller, Hans-Jörg, W-3000 Hannover 61 (DE)
(74) Vertreter: Lauer, Dieter, Dr.

(56) Entgegenhaltungen:
- EP-A- 0 216 272
- EP-A- 0 294 711
- DE-A- 3 228 477

## Beschreibung

Die Erfindung bezieht sich auf ein Verfahren zur Herstellung hydrolysestabiler Enzymträger auf anorganischer Basis und mit organischen Enzymbindungsstellen, die aus einem rundlichen, makropermeablen Agglomerat von Primärpartikeln, die zu einer maulbeerartigen Substruktur angeordnet sind, bestehen, auf den hergestellten Enzymträger und die durch Immobilisierung von Enzymen an diesen Träger hergestellten Trägerkatalysatoren.

Im Stand der Technik ist es bekannt, daß sich auf Basis von porösen Siliciumdioxid- bzw. Aluminiumoxidträgermaterialien Trägerkatalysatoren (mit Enzym belegter Träger) mit hohen Enzymdichten herstellen lassen, die bei ihrem Einsatz einen kontrollierten Stoffaustausch und hohe Produktivität zeigen. Sowohl die Trägermaterialien aus Siliciumdioxid als auch aus Aluminiumoxid sind inkompressibel und können mit definierter Porenstruktur und Porenverteilung sowie hohem Porenvolumen hergestellt werden.

Siliciumdioxid als Trägermaterial ist zwar ein preiswertes und gut zugängliches Ausgangsmaterial, welches sich leicht mit den für Enzymträger gewünschten Eigenschaften (z.B. Porosität, Kornfraktion, Schüttgewicht etc.) herstellen läßt und welches unter den in der industriellen Praxis auftretenden hydrodynamischen Belastungen auch abriebfest ist. Siliciumdioxid-Trägermaterialien sind jedoch mit dem Nachteil behaftet, daß sie im neutralen bis schwach alkalischen pH-Bereich nicht hydrolysestabil sind. Diese negative Eigenschaft führt in der Praxis zu Problemen, wenn auf dem Siliciumdioxid-Trägermaterial Enzyme immobilisiert werden, deren Aktivitätsoptimum ein Substrat mit neutralem bis schwach alkalischem pH-Wert erfordert. Ein Beispiel für ein solches Enzym ist Glucoseisomerase, die an Siliciumdioxid-Träger immobilisiert zur teilweisen Umwandlung von Glucosesubstrat in ein Gemisch aus Glucose und Fructose (hochfructosehaltiger Sirup, HFS) industriell eingesetzt wird. An diesem Beispiel eines Trägerkatalysators treten die Nachteile des Siliciumdioxid-Trägermaterials deutlich hervor, da auch das mit Enzym belegte Siliciumdioxid während der Umsetzung des Glucosesubstrats (Isomerisierung) einer deutlichen Hydrolyse unterliegt. Die Hydrolyse des Trägers bewirkt einen mit Enzymverlust verbundenen, ausgeprägten Verlust an Trägermasse, wodurch die Standzeit von Isomerisierungsreaktoren stark reduziert werden kann. Im Extremfall kann dadurch sogar eine Verstopfung des Enzymbettes im Reaktor auftreten, wobei die gesamte noch im Reaktor vorhandene Restaktivität des Trägerkatalysators (unter Umständen bis zu 40 % der eingesetzten Aktivität) verlorengehen kann.

Träger aus Aluminiumoxid sind zwar hydrolysestabil, jedoch ist deren Herstellverfahren aufwendig. Das Ausgangsmaterial muß zunächst bei Temperaturen, die deutlich über 1000 °C liegen, kalziniert, danach zum Erhalt der gewünschten Kornfraktion gebrochen und gesiebt werden. Beim Brechen und Sieben treten aufgrund der kreidigen Struktur des Materials hohe Materialverluste auf und die hierbei erhaltene, verwendbare Aluminiumoxidträger-Fraktion behält auch nach zusätzlichen Behandlungsmaßnahmen und anschließender Enzymbelegung (Trägerkatalysator) die unerwünschte Eigenschaft des Abkreidens bei. Diese negative Eigenschaft ist für den Einsatz eines solchen Trägerkatalysators in der industriellen Praxis nachteilig, da unter den dort im Reaktor gegebenen hydrodynamischen Bedingungen Abrieb, Enzymverluste und Reaktorverstopfungen auftreten.

Es bestand daher die Aufgabe, einen neuen, aus verfügbaren Materialien leicht herstellbaren Enzymträger auf anorganischer Basis bereitzustellen, der die Nachteile des Standes der Technik überwindet, und ein Verfahren zu dessen Herstellung anzugeben. Eine weitere Aufgabe bestand in der Bereitstellung trägergebundener Enzyme (Trägerkatalysatoren) unter Verwendung des neuen Enzymträgers.

Die Aufgaben werden gelöst durch das erfindungsgemäße Verfahren zur Herstellung der neuen Enzymträger, durch die neuen Enzymträger sowie die neuen trägergebundenen Enzyme.

Die Erfindung betrifft ein Verfahren zur Herstellung von Enzymträgern auf anorganischer Basis und mit organischen Enzymbindungsstellen, bestehend aus einem rundlichen, makropermeablen Agglomerat von Primärpartikeln, die zu einer maulbeerartigen Substruktur angeordnet sind, wobei sich das Verfahren dadurch auszeichnet, daß
a) man kugelförmige Primärpartikel aus mikroporösem α-Aluminiumoxidhydroxid der Formel AlO(OH) unter Verwendung eines organischen, vernetzungsfähige reaktive funktionelle Gruppen aufweisenden Binders so zu einer maulbeerartigen Substruktur aus zusammengelagerten Primärpartikeln granuliert, daß die Individualität der Primärpartikel darin im wesentlichen erhalten bleibt,
b) man das in a) erhaltene Agglomerat in seiner Form und Struktur verfestigt, indem man den das Agglomerat durchsetzenden Binder mit einem, mit den reaktiven funktionellen Gruppen des Binders reagierenden organischen Härter zu einem dreidimensionalen, die maulbeerartige Substruktur durchdringenden Netzwerk aus organischem Polykondensat so umsetzt, daß
c) ein Teil der reaktiven Gruppen des organischen Binders und/oder des organischen Härters im Netzwerk bildenden Polykondensat als freie funktionelle Gruppen zur Enzymbindung erhalten bleibt.

Als Primärpartikel für den erfindungsgemäßen Enzymträger wird ein feinkörniges, kugelförmiges, mikroporöses α-Aluminiumoxidhydroxid der Formel AlO(OH) verwendet. Es ist ein preiswertes, hydrolysestabiles Ausgangsmaterial, welches kommerziell verfügbar ist bzw. leicht nach an sich bekannten Verfahren hergestellt werden kann. Das α-Aluminiumoxidhydroxid zeichnet sich durch hohe chemische Reinheit aus und besitzt im allgemeinen folgende typische, jedoch nicht einschränkend zu verstehende, Kenndaten:

| | |
|---|---|
| Aluminiumoxid-Gehalt: | mindestens 75 Gew.-%, im allgemeinen etwa 79 Gew.-%; |
| Korngrößenverteilung: | < 25 »m: maximal 40 %, |
| | < 45 »m: maximal 60 %; |
| | > 90 »m: maximal 20 %; |
| Schüttgewicht: | 0,45 bis 0,65 g/ml; |
| Porenvolumen (PV): | mindestens 0,6 ml/g, im allgemeinen etwa 0,75 ml/g; |
| häufigster Porendurchmesser (HPD): | 700 bis 900 Angström; |
| spezifische Oberfläche: | mindestens 85 m²/g, im allgemeinen etwa 98 m²/g. |

Nach den vorstehenden Porositätsdaten (PV, HPD) ist α-Aluminiumoxidhydroxid als Trägermaterial für immobilisierte Enzyme sehr gut geeignet. Als technisch verwendbarer Enzymträger kommt dieses Material als solches jedoch nicht in Frage, da es um etwa eine Größenordnung zu kleine Partikelabmessungen besitzt. Es kann verfahrensbedingt (Sprühtrocknung) auch nicht mit vertretbarer Ausbeute in der erforderlichen Korngröße erzeugt werden.

Erfindungsgemäß wird dem Mangel der zu kleinen Partikelabmessungen dadurch abgeholfen, daß die α-AlO(OH)-Primärpartikel unter Verwendung eines organischen Binders, der vernetzungsfähige reaktive funktionelle Gruppen aufweist, zu einem rundlichen Agglomerat von technisch geeigneter Korngröße granuliert wird und die Form und die Struktur des Agglomerates durch Umsetzung des organischen Binders mit einem organischen Härter, der mit den reaktiven funktionellen Gruppen des Binders reagieren kann, stabilisiert wird. Die erhaltenen rundlichen Agglomerate bestehen aus einem Haufwerk von α-AlO(OH)-Primärpartikeln, die durch die Granulation nur so dicht aneinandergelagert und miteinander verbunden sind, daß das Agglomerat makropermeabel bleibt. Die Makropermeabilität des Agglomerates gewährleistet dem zu immobilisierenden Enzym und dessen Substrat ungehinderten Zutritt zum Mikroporensystem der Primärpartikel. Hierdurch sind günstige Enzymaufnahmeeigenschaften sowie ein günstiger Stoffaustausch gewährleistet. Das Agglomerat aus maulbeerartig angeordneten Primärpartikeln wird in seiner Form und Struktur durch das Polykondensat aus dem organischen Binder und dem als Vernetzungsmittel dienenden organischen Härter so stabilisiert, daß ein abriebfester Enzymträger resultiert, dessen Permeabilität nicht beeinträchtigt ist.

Das Ergebnis des erfindungsgemäßen Verfahrens ist überraschend, da es durch gewöhnliche Granulation der α-AlO(OH)-Primärpartikel mit beispielsweise nur Wasser als Befeuchtungsmittel nicht gelingt, Granulate aus α-AlO(OH) herzustellen, die als Enzymträger geeignet sind. Zwar lassen sich nach dieser gewöhnlichen Granulationsmethode auch Granulate mit den benötigten Partikelabmessungen erzeugen, die ebenfalls aus einem Haufwerk von Primärpartikeln bestehen, jedoch sind diese Granulate nicht abriebfest und nicht makropermeabel. Die Abriebfestigkeit kann nicht durch nachträgliches Aufbringen des organischen Binders auf die, nur durch Granulation mit Wasser gewonnenen Agglomerate hergestellt werden. Im Falle der Granulation nur mit Wasser werden die interpartikulären Hohlräume zwischen den agglomerierten Primärpartikeln durch den sich bei dieser Verfahrensweise bildenden Abrieb von AlO(OH) verstopft und es wird daher keine maulbeerartige Struktur erzielt und die Makropermeabilität des Agglomerates geht verloren. Wird auf ein derartig verklebtes Agglomerat nachträglich organischer Binder aufgebracht, so kann dieser das Agglomerat nur noch unzureichend durchdringen. Die Form und die Struktur des Agglomerates kann durch die Vernetzung des organischen Binders mit dem organischen Härter nur unzureichend stabilisiert werden. Solche nicht erfindungsgemäßen Agglomerate neigen zum Auseinanderbrechen und sind nicht abriebfest.

Darüber hinaus schneiden diese nicht erfindungsgemäßen Agglomerate auch hinsichtlich Enzymaufnahme und Stoffaustausch als Trägerkatalysatoren bei der Umsetzung eines Substrates im Gegensatz zu den erfindungsgemäßen Trägern so ungünstig ab, daß sie für einen technischen Einsatz nicht geeignet Sind.

Die erfindungsgemäßen Vorteile werden durch Verwendung eines organischen Binders bei der Granulation der Primärpartikel, wobei dieser Binder vernetzungsfähige reaktive funktionelle Gruppen aufweist, und durch die Vernetzung des Binders mit einem organischen Härter, der mit den reaktiven funktionellen Gruppen des Binders reagieren kann, erreicht. Durch die Vernetzung des organischen Binders mit dem organischen Härter werden nicht alle funktionellen Gruppen des Binders und/oder des Härters verbraucht, so daß das zur Verfestigung des erfindungsgemäßen Enzymträgers dienende Netzwerk aus dem organischen Binder/Härter-Polykondensat in vorteilhafter Weise gleichzeitig freie organische funktionelle Gruppen zur Enzymbindung zur Verfügung stellt.

Als organische Binder sind insbesondere Polyimine zweckmäßig. Bevorzugt wird Polyethylenimin als organischer Binder verwendet.

Als organische Härter sind insbesondere Dialdehyde oder Epoxidverbindungen zweckmäßig. Ein bevorzugt verwendeter Dialdehyd ist Glutardialdehyd; bevorzugt verwendete Epoxidverbindungen sind epoxyfunktionelle Siliconharze, z.B. aus 3-Glycidoxypropyltriethoxysilan und aliphatische Di- und Triepoxide (z.B. Epon 812, Serva Heidelberg, Nr. 21045). Die verwendbaren epoxyfunktionellen Alkoxysilane, z.B. das 3-Glycidoxypropyltriethoxysilan, werden für die Verwendung als Härter zunächst durch Hydrolyse unter Einfluß sauerer Katalysatoren mit Wasser oder wasserhaltigen organischen Lösungsmitteln in das aktive Trisilanol überführt. Die Hydrolyse erfolgt rasch, wobei auch Kondensation des primär gebildeten Silantriols (gleichfalls säurekatalysiert) stattfindet; die Epoxyfunktion bleibt jedoch erhalten. Derart erhaltene Stammlösungen aus epoxyfunktionellem Siliconharz werden z.B. mit Wasser oder Alkohol auf die gewünschte Konzentration verdünnt und für die Vernetzung des organischen Binders verwendet.

Durch die vorstehend beschriebenen organischen Binder bzw. Härter werden als Enzymbindungsstellen einerseits primäre, sekundäre und tertiäre Aminogruppen (aus dem Binder) und andererseits Aldehydgruppen oder Epoxidgruppen (aus dem Härter), die nicht an der Vernetzungsreaktion zwischen Binder und Härter beteiligt sind, bereitgestellt.

Der Granulationsschritt des erfindungsgemäßen Verfahrens kann in an sich üblichen Granulationseinrichtungen wie z.B. Mischern oder Drehtellern durchgeführt werden. Alternativ kann die Granulation auch in einem Wirbelbett als Sprühgranulationsverfahren durchgeführt werden.

Hierzu wird beispielsweise α-Aluminiumoxidhydroxid der oben beschriebenen Qualität (bspw. Aluminiumoxidhydrate der Firma Condea Chemie, Brunsbüttel; z. B. Pural^{R}) in einem geeigneten Drehteller vorgelegt und kontinuierlich über einen kurzen Zeitraum, zweckmäßigerweise etwa 5 bis 20 Minuten, mit einer wäßrigen Lösung von Polyethylenimin (PEI) besprüht. Hierbei werden zusammengelagerte, makropermeable Agglomerate der α-AlO(OH)-Primärpartikel erhalten, die dann nach Korngröße klassiert werden. Die gewünschte Agglomeratkornfraktion (Gutkorn) wird abgetrennt, die verbleibende Überkornfraktion wird zerkleinert und gemeinsam mit der Unterkornfraktion dem Drehteller wieder zugegeben. Unter Beigabe von frischem Primärkorn und weiterem Besprühen mit Polyethyleniminlösung wird der Granulationsprozeß fortgeführt, bis erneut hinreichend Agglomerat gebildet ist und Gutkorn abgetrennt werden kann. Das Verfahren wird solange fortgesetzt, bis die gewünschte Menge an Gutkorn erzeugt ist; die bei der Klassierung des Agglomerates verbleibenden kleinen Restmengen an Über- bzw. Unterkorn können zwischengelagert und als Startmaterial für spätere Produktionschargen verwendet werden.

Der Prozeß muß nicht quasi-kontinuierlich geführt werden, es ist auch möglich, laufend von frischem Primärkorn auszugehen und die sich dabei ansammelnden Nebenfraktionen später getrennt oder gemeinsam weiterzuverarbeiten.

Die Menge des auf die Primärpartikel aufgebrachten Polyimins kann durch Variation der Konzentration der versprühten Polyimin-Lösung, die Sprühdauer und den Durchsatz während des Sprühens gesteuert werden. In einer bevorzugten Ausgestaltung des erfindungsgemäßen Verfahrens wird eine wäßrige Lösung des Polyimins, vorzugsweise des Polyethylenimins, in einer Konzentration von 5 bis 15 Gew.-% bei Auftragungszeiten von 5 bis 20 Minuten eingesetzt. Besonders bevorzugt sind wäßrige Lösungen des Polyimins mit einer Konzentration von etwa 10 Gew.-% an Polyimin, die zweckmäßigerweise mit Sprühzeiten von etwa 10 Minuten angewendet werden. Der Durchsatz an Polyiminlösung wird dabei bevorzugt so bemessen, daß ca. 0,5 kg Polyiminlösung pro 1 kg α-Aluminiumoxidhydroxid aufgetragen werden. Derart hergestellte erfindungsgemäße Enzymträger weisen in der klassierten Gutkorn-Fraktion Polyimingehalte von 3 bis 7 Gew.-%, vorzugsweise von 4 bis 6 Gew.-% (bezogen auf das Gesamtgewicht des Enzymträgers) auf.

Die erzeugte Gutkornfraktion des Agglomerates wird zweckmäßig im Drehteller unter weiterem Besprühen mit Polyethyleniminlösung über einen kurzen Zeitraum, vorzugsweise etwa 3 bis 5 Minuten, nachgerollt.

Danach wird das erhaltene Gutkorn bei Temperaturen von etwa 60 °C bis zu einem Restfeuchtegehalt ≦ 5 Gew.-% getrocknet. Hierzu kann an sich jedes geeignete kontinuierliche oder diskontinuierliche Trockenverfahren eingesetzt werden. Die getrockneten, rundlichen Agglomerate, aus den α-AlO(OH)-Primärpartikel, die zu einer maulbeerartigen, makropermeablen Substruktur angeordnet sind, werden anschließend in Form und Struktur durch Kontakt mit aminreaktiven Vernetzungsmitteln (Härter) stabilisiert. Dazu wird das Agglomeratmaterial z.B. in eine Glutardialdehyd enthaltende Phosphatpufferlösung gegeben oder eine Lösung von epoxyfunktionellen Siliconharzen oder aliphatischen Di- oder Triepoxiden aufgetränkt. Dieser Verfahrensschritt ist unkritisch. Nach hinreichender Reaktionszeit wird das durch Vernetzung stabilisierte Agglomerat gewaschen, um Restanteile des Vernetzungsmittels zu entfernen.

Es liegt hiernach der fertige Enzymträger vor, der nachfolgend gegebenenfalls feucht oder auch getrocknet zur Enzymfixierung eingesetzt werden kann.

Die Erfindung betrifft weiterhin die Enzymträger, die nach den vorstehend beschriebenen Verfahren hergestellt werden können. Diese Gegenstände der Erfindung sind Enzymträger auf anorganischer Basis und mit organischen funktionellen Enzymbindungsstellen, bestehend aus einem rundlichen, makropermeablen Agglomerat von Primärpartikeln, die zu einer maulbeerartigen Substruktur angeordnet sind, wobei sich dieser Enzymträger dadurch auszeichnet, daß
a) die maulbeerartige Substruktur aus zusammengelagerten, kugelförmigen Primärpartikeln von mikroporösem α-Aluminiumoxidhydroxid der Formel AlO(OH) gebildet ist und die Individualität der Primärpartikel darin im wesentlichen erhalten ist,
b) das Agglomerat in seiner Form und Struktur durch ein dreidimensionales, die maulbeerartige Substruktur durchdringendes Netzwerk, bestehend aus einem organischen Polykondensat, verfestigt ist, und
c) das Netzwerk bildende organische Polykondensat noch freie organische funktionelle Gruppen als Bindungsstellen für Enzyme besitzt.

Die Erfindung betrifft auch trägergebundene Enzyme (Trägerkatalysatoren), die sich dadurch auszeichnen, daß sie an den vorstehend beschriebenen erfindungsgemäßen Enzymträger gebunden sind. Als besonders bevorzugt ist die Anwendung des erfindungsgemäßen Enzymträgers für die Immobilisierung von Glucoseisomerase zu nennen. Die Vorteile des erfindungsgemäßen Enzymträgers kommen bei diesem Einsatzzweck voll zur Geltung.

Die aktive Komponente, d.h. Enzyme wie Glucoseisomerase, können direkt auf den erfindungsgemäßen Enzymträger aufgebracht werden. Eine Vorbehandlung des Trägers zur Erzeugung von Enzymbindungsstellen ist nicht mehr nötig, da zur Immobilisierung benötigten Bindungsstellen bereits während der Herstellung des erfindungsgemäßen Enzymträgers über das Netzwerk-bildende organische Polykondensat in ausreichender Zahl einerseits z.B. durch nicht umgesetzte primäre sowie sekundäre und tertiäre Aminogruppen aus dem Binder und andererseits z.B. durch nicht umgesetzte Aldehyd- oder Epoxidgruppen aus dem Härter bereitgestellt werden. Die Träger werden daher in einfacher Weise mit einer an sich üblichen flüssigen Enzymzubereitung kontaktiert, wobei das Enzym an die genannten Bindungsstellen fixiert wird. Anschließend kann das auf dem Enzymträger fixierte Enzym noch in an sich bekannter Weise einer Quervernetzung unterworfen werden.

Durch die Erfindung werden in vorteilhafter Weise neue Enzymträger zur Verfügung gestellt, die sehr einfach und kostengünstig hergestellt werden können. Die erfindungsgemäßen Enzymträger besitzen eine in strömungstechnischer Hinsicht vorteilhafte rundliche Gestalt, sie sind hydrolysestabil, abriebfest und dennoch makropermeabel, wodurch ein günstiger Stoffaustausch gewährleistet ist. Der erfindungsgemäße Enzymträger zeichnet sich somit gegenüber den gebrochenen SiO₂-Trägern (Splitt) des Standes der Technik aus, die nicht hydrolysestabil sind und formbedingt strömungstechnische Nachteile aufweisen. Die erfindungsgemäßen Enzymträger zeichnen sich auch gegenüber gebrochenen Al₂O₃-Trägern (Splitt) des Standes der Technik aus, da letztere wegen der kreidigen Struktur des porösen Aluminiumoxides nur unter hohen Ausbeuteverlusten gefertigt werden können, nicht abriebfest sind und gleichfalls aus strömungstechnischer Sicht erhebliche Nachteile aufweisen. Die Vorteile der erfindungsgemäßen Enzymträger kommen auch in den daraus hergestellten Trägerkatalysatoren voll zur Geltung.

Die nachfolgenden Beispiele sollen die Erfindung weiter erläutern, ohne sie jedoch in ihrem Umfang zu beschränken.

### I. Herstellung erfindungsgemäßer Träger

Für die nachfolgend hergestellten erfindungsgemäßen Träger wurde kugelförmiges, durch Sprühtrocknung hergestelltes α-Aluminiumoxidhydroxid der Formel AlO(OH) mit folgenden typischen Kenndaten eingesetzt:

| | |
|---|---|
| Aluminiumoxid-Gehalt: | 79 Gew.-% |
| Korngrößenverteilung: | <25 »m: 12,9 % |
| | <45 »m: 57,6 % |
| | <90 »m: 23,5 % |
| | >90 »m: 6,0 % |
| Schüttgewicht: | 0,52 g/ml |
| Porenvolumen (PV): | 0,75 ml/g |
| Häufigster Porendurchmesser (HPD): | 742 Angström |
| Sezifische Oberfläche: | 98 m²/g |

### Träger Nr. 1

3 kg des Aluminiumoxidhydroxides der Formel AlO(OH) wurden in einem Drehteller vorgelegt und mit einer 9,7 gew.-%igen Polyethylenimin-Lösung über einen Zeitraum von 12 Minuten besprüht. Man erhielt als Gutkornfraktion ein Agglomerat mit Partikeldurchmessern von 0,3 bis 0,6 mm. Der Polyethylenimingehalt des Gutkornes betrug 5,9 Gew.-% (C/N-Analyse). Nachfolgend wurden das Agglomerat bei ca. 60 °C bis zu einem Restfeuchtegehalt ≦ 5 Gew.-% getrocknet. Das getrocknete Material wurde anschließend mit einer 2,5 Gew.-%igen Lösung von Glutardialdehyd in 0,05 molarem Phosphatpuffer mit einem pH-Wert von 6,0 (3 ml Lösung/g Träger) über einen Zeitraum von 1 h vernetzt. Das erhaltene, vernetzte Agglomerat wurde von Glutardialdehyd-Resten freigewaschen und bei etwa 60 °C getrocknet.

### Träger Nr. 2

Die Herstellung erfolgte wie bei Träger Nr. 1, jedoch betrug die Sprühzeit 8 Minuten und es wurden Agglomerate mit Polyethylenimin-Gehalten von 4,1 Gew.-% (C/N-Analyse) erhalten.

### Träger Nr. 3

Analog zur Herstellung des Trägers Nr. 1 wurde zunächst α-Aluminiumoxidhydroxid über einen Zeitraum von 14 Minuten mit Polyethylenimin-Lösung besprüht. Man erhielt Agglomerate mit einem Polyethylenimin-Gehalt von 6,8 Gew.-% (C/N-Analyse). Das erhaltene Agglomerat wurde bei 60 °C getrocknet und das getrocknete Material anschließend durch Auftränken von epoxyfunktionellem Silikonharz (75 mg Harz/g Träger) aus ethanolischer Lösung vernetzt. Der durch Vernetzung stabilisierte Träger wurde anschließend bei 60 °C und nachfolgend bei 110 °C getrocknet.

Die zur Vernetzung eingesetzte ethanolische Lösung von epoxyfunktionellem Silikonharz wurde wie folgt hergesellt: Zu 70 ml 3-Glycidoxypropyltriethoxysilan wurden 30 ml Wasser (das pro l 300 mg 60 %ige Perchlorsäure enthält) gegeben und dieser Ansatz über 30 Min. kräftig gerührt. Nach 1 h Standzeit wurde durch Verdünnen einer Teilmenge mit Wasser auf das zehnfache Volumen geprüft, ob alles Ethoxysilan zum Silantriol durchhydrolysiert ist (keine Trübung). Der homogene Ansatz wurde dann mit Ethanol (90 %ig) auf 7,5 Vol.-% Wirkstoffgehalt verdünnt und von dieser Lösung 1 ml/g Träger aufgetränkt.

### Träger Nr. 4

Zunächst wurde analog zur Herstellung des Trägers Nr. 1 α-Aluminiumoxidhydroxid mit Polyethylenimin-Lösung zu einem Agglomerat granuliert. Anschließend wurde das Agglomerat durch Tränken mit einer Lösung von aliphatischen Di- und Triepoxiden in Dioxan vernetzt (70 mg Epoxidverbindung/g Träger). Man ließ 24 h bei 20 °C und 1 h bei 90 °C reagieren. Danach wurde das durch Quervernetzung stabilisierte Agglomerat durch Waschen mit Aceton Epoxid- und Dioxan-frei gewaschen. Abschließend wurde bei 60 °C und nachfolgend bei 110 °C getrocknet.

### Träger Nr. 5

2 kg α-Aluminiumoxidhydroxid der Formel AlO(OH) wurden in einem Fließbett-Sprühgranulator mit einer 4,25 gew.-%igen wäßrigen Polyethylenimin-Lösung granuliert. Der Polyethylenimin-Gehalt des erhaltenen Agglomerates betrug 5,8 Gew.-% (C/N-Analyse). Das Agglomerat wurde analog der Herstellung des Trägers Nr. 1 durch Vernetzung mit Glutardialdehyd stabilisiert.

### II. Herstellung von nichterfindungsgemäßen Vergleichsträgern

### Vergleichsträger Nr. V1

Kugelförmiges α-Aluminiumoxidhydroxid der Formel AlO(OH) wurde zunächst analog Beispiel I. Nr. 1 zu einem Agglomerat granuliert. Zum Befeuchten während der Granulation wurde jedoch anstelle von wäßriger Polyethylenimin-Lösung ausschließlich Wasser verwendet. Auf die erhaltene, getrocknete Gutkornfraktion wurde dann nachträglich 5 Gew.-% Polyethylenimin aus wäßriger Lösung aufgetränkt und das Material wie unter I. Nr. 1 angegeben mit Glutardialdehyd vernetzt.

### Vergleichsträger Nr. V2

Auf Strangpresslinge aus α-Aluminiumoxidhydroxid der Formel AlO(OH) mit einem Durchmesser von 0,75 mm wurden 5 Gew.-% Polyethylenimin aus wäßriger Lösung aufgetränkt. Die Vernetzung erfolgte
a) mit Glutardialdehyd wie unter Beispiel I. Nr. 1 angegeben;
b) mit epoxyfunktionellem Silikonharz, wie unter Beispiel I. Nr. 3 angegeben;
c) mit aliphatischen Di- und Triepoxiden, wie unter Beispiel I. Nr. 4 angegeben.

### Vergleichsträger Nr. V3

Auf ein vom Hersteller (Firma Condea Chemie) bezogenes, vorgefertigtes Granulat aus α-Aluminiumoxidhydroxid mit Partikeldurchmessern von 0,5 bis 1 mm wurden 4 Gew.-% Polyethylenimin aus wäßriger Lösung aufgetränkt. Die Vernetzung erfolgte mit Glutardialdehyd wie unter Beispiel I. Nr. 1 angegeben.

### III. Herstellung von Trägerkatalysatoren

Die unter I. hergestellten erfindungsgemäßen Träger und die unter II. hergestellten Vergleichsträger wurden mit 0,05 molarem Natriumacetatpuffer (pH 5,7) equilibriert. Nach Entfernen des überschüssigen Puffers wurden 14000 U Glucoseisomerase (aus Streptomyces rubiginosus) in einem Gesamtflüssigkeitsvolumen von 3 ml eines 0,05 molaren Natriumacetatpuffers pro 1 g Träger zugegeben (1 U ist definiert als die Enzymmenge, die unter Anfangsreaktionsbedingungen Glucose zu Fructose mit einer Rate von 1 mg/ml·h umsetzt; 2 molares Glucosesubstrat in 0,1 molarem Maleatpuffer (pH 7) mit 5 mmol Mg²⁺ und 0,125 mmol Co²⁺, 60 °C). Die Ansätze wurden unter gelegentlichem Umschwenken 20 h stehengelassen und der pH-Wert dabei auf ≦ pH 6,5 gehalten. Danach wurde mit 0,05 molarem Natriumacetatpuffer gewaschen, um gegebenenfalls nicht gebundenes Enzym zu entfernen. Der überschüssige Waschpuffer wurde abgesaugt und anschließend 3 ml einer 2,5 gew.-%igen Lösung von GDA in 0,05 molarem Natriumacetatpuffer pro 1 g Träger aufgegeben. Der Ansatz wurde über 30 Minuten gelegentlich geschwenkt, die GDA-Lösung danach abgesaugt und das Material mit 0,05 molarem Natriumacetatpuffer und anschließend mit destilliertem Wasser gewaschen, um überschüssigen GDA zu entfernen. Nach Absaugen überschüssiger Feuchte wurden 2 ml einer 4 gew.-%igen wäßrigen Lösung von Polyethylenimin, die mit Schwefelsäure auf pH 5,0 eingestellt war, pro 1 g Träger aufgegeben. Nach einer Standzeit von 20 h, während der gelegentlich umgeschwenkt wurde, wurde die überschüssige Polyethyleniminlösung abgetrennt und mit Wasser gewaschen. Nach Austausch des Waschwassers gegen Konservierungsstoffe enthaltenden 0,05 molaren Phosphatpuffer (pH 6) waren die Trägerkatalysatoren lagerfähig.

Die Enzymaufnahme der in dieser Weise hergestellten Trägerkatalysatoren wurde aus der Differenz der Aktivitäten des Immobilisierungsansatzes vor und nach der Enzymbelegung des Trägers bestimmt. Die Enzymaufnahme lag typischerweise zwischen 96 und 100 % der angebotenen Enzymmenge.

Die hergestellten erfindungsgemäßen Trägerkatalysatoren und deren Eigenschaften sind in Tabelle I, die hergestellten Vergleichsträgerkatalysatoren in Tabelle II zusammengestellt.

### IV. Eigenschaften erfindungsgemäßer Trägerkatalysatoren im Vergleich zu nicht erfindungsgemäßen Trägerkatalysatoren

Zur Qualitätsbeurteilung der erfindungsgemäßen Enzymträger wurden die Eignung als Trägerkatalysator für Glucoseisomerase (Enzymaufnahme, Anfangsraumgeschwindigkeit, Produktivität) und die Abriebfestigkeit herangezogen.

### a) Abriebfestigkeit

Die Abriebfestigkeit wurde in einem Streßtest geprüft, der hydrodynamische Belastungen, denen das Material unter Praxisbedingungen ausgesetzt sein kann, simuliert. Dazu wurden 1 g Träger oder Trägerkatalysator zusammen mit 4 g Wasser in ein zylindrisches Gefäß (Durchmesser 20 mm) gegeben und auf einem Whirlmixer bei voller Leistung (1400 Min⁻¹ unter Belastung) gestreßt. Als abriebfest wurde Trägermaterial eingestuft, das nach 2 Minuten keine Trübung im Überstand der Suspension zeigte. Die hergestellten erfindungsgemäßen Enzymträger bzw. Trägerkatalysatoren erwiesen sich durchweg als abriebfest.

### b) Isomerisierung

Die erfindungsgemäßen, abriebfesten Trägerkatalysatoren und die nichterfindungsgemäßen Vergleichspräparate wurden unter Isomerisierungsbedingungen, die den Gegebenheiten der Praxis entsprechen, auf ihre Leistungsfähigkeit hin untersucht. Hierzu wurden die Trägerkatalysatoren in einen temperierten Säulenreaktor eingebaut und vortemperiertes Substrat mit einer Dosierpumpe durch das Trägerkatalysatorbett geleitet. Der Durchsatz der Substratlösung wurde so eingestellt, daß ein Isomerisierungsgrad von 46,5 % erreicht und über die gesamte Versuchsdauer aufrechterhalten wurde. Die Reaktionstemperatur betrug 60 °C. Das eingesetzte Substrat hatte folgende Zusammensetzung:
45 Gew.-% Glucose in wäßriger Lösung,
120 ppm Mg (II) als MgSO₄,
300 ppm SO₂ als Na₂SO₃,
pH-Wert 7,5.

### c) Ergebnisse

Die Ergebnisse sind in den Tabellen I und II zusammengestellt. Die Enzymaufnahme ist angegeben in U/ml Katalysator, wobei 1 U definiert ist als die Enzymmenge, die unter Anfangsreaktionsbedingungen Glucose in Fructose mit einer Rate von 1 mg/ml·h umsetzt (2 molares Glucosesubstrat in 0,1-molarem Maleatpuffer pH 7 mit 5 mmol Mg²⁺, 0,125 mmol Co²⁺, 60 °C).

Als Maß für die Trägerkatalysator-Aktivität ist die Anfangsraumgeschwindigkeit (RG = ml Substrat/ml Katalysator·h) aufgeführt.

Soweit die Produktivität im Langzeittest geprüft wurde, ist diese im Zusammenhang mit der noch vorhandenen Restaktivität in t_{HFS}(TS)/l Trägerkatalysator angegeben.

Zu Versuchsende wurde die Katalysatorfüllung jeweils ausgebaut, mit Wasser zuckerfrei gewaschen, getrocknet und die Restmasse durch Wägen bestimmt.

### Zu Tabelle I

Die Ergebnisse in Tabelle I zeigen, daß die erfindungsgemäßen Enzymträger hervorragend zur Herstellung von Trägerkatalysatoren geeignet sind. Gegenüber dem als Vergleich angegebenen porösen Siliciumdioxid-Träger gemäß Stand der Technik wurden hinsichtlich Enzymaufnahme und Enzymnutzbarkeit im immobilisierten Zustand gleichwertige, hohe Werte erreicht; hinsichtlich Standverhalten (vergleiche t_{50%}, t_{20%}) und Produktivität ist das erfindungsgemäße Trägermaterial deutlich überlegen, da es im Glucosesubstrat hydrolysestabil ist und sich nicht wie der Siliciumdioxid-Träger des Standes der Technik allmählich auflöst (siehe Katalysatorrestmassen). Gegenüber Aluminiumoxid-Trägern des Standes der Technik mit Halbwertzeiten von etwa t_{50%}= 2.500 h wurden vergleichbare, teilweise sogar höhere Halbwertzeiten von bis zu etwa t_{50%}= 3.000 h mit den erfindungsgemäßen Enzymträgern erreicht. Die erfindungsgemäßen Enzymträger besitzen gegenüber den Aluminiumoxid-Trägern des Standes der Technik jedoch den zusätzlichen Vorteil, daß sie wesentlich kostengünstiger (50 % der Kosten eines Aluminimoxid-Trägers) gefertig werden können und sie sind nicht mit dem Nachteil behaftet, unter hydrodynamischem Streß, dem sie bei ihrer Verwendung ausgesetzt sind, laufend abzukreiden.

### Zu Tabelle II

Die unter II. hergestellten bzw. schon ausgeformt vom Hersteller bezogenen Vergleichsträger aus α-Aluminiumoxidhydroxid schneiden hinsichtlich Enzymaufnahme, erzielbarer Raumgeschwindigkeit und Abriebverhalten (im Streßtest nicht abriebfest) gegenüber den erfindungsgemäßen Trägern so ungünstig ab, daß sie mangels technischer Eignung nicht im Langzeittest (Produktivität) geprüft wurden.

Die geringen Enzymaufnahmen und geringen Raumgeschwindigkeiten lassen erkennen, daß die Vergleichspräparate hinreichend makropermeable Substrukturen, die den erforderlichen Stoffaustausch bei der Enzymbelegung und Substratumsetzung gewährleisten, nicht aufweisen.

Durch mikroskopische Kontrolle wurde festgestellt, daß beim Vergleichsträger II. Nr. V1 zwar auch ein Haufwerk aus Primärkorn von α-Aluminiumoxidhydroxid vorliegt, dieses Haufwerk aber durch Abrieb, der während der Granulation beim Besprühen mit ausschließlich Wasser (anstelle wäßriger Polyethylenimin-Lösung) entsteht, weitgehend verklebt wird, so daß dessen Permeabilität verloren geht. Das hier erst nach Granulation des Primärkorns aufgebrachte Polyethylenimin kann das Haufwerk infolgedessen nur unzureichend durchdringen. Das Granulat ist nach Vernetzung in Form und Struktur nur unzureichend stabilisiert (Abrieb im Streßtest).

Die Vergleichsträger II. Nr. V2 und II Nr. V3 (die auf bereits ausgeformten, kommerziell erhältlichen Trägern aus α-Aluminiumoxidhydroxid beruhen) wiesen bei mikroskopischer Überprüfung keine Substrukturen aus Primärkorn auf. Offensichtlich wurde bei deren konventioneller Formgebung (Verpressen, Granulieren) die makropermeable Substruktur vollständig zerstört. Für die Wirkmöglichkeit des auch hier erst auf die fertigen Formkörper aufgebrachten organischen Binders und des Vernetzungsmittels (Polyethylenimin/Glutardialdehyd etc.) gilt das vorstehend zum Vergleichsträger II. Nr. V1 ausgeführte analog; die Vergleichsträger II. Nr. V2 und II. Nr. V3 reiben im Streßtest ab.

Insgesamt hebt sich damit der erfindungsgemäße Enzymträger entscheidend von Vergleichsmaterialien des Standes der Technik ab; erfindungsgemäße Enzymträger besitzen insbesondere die Vorteile: Makropermeabilität (günstiger Stoffaustausch), sie sind dennoch abriebfest, besitzen eine rundliche (weitgehend kugelförmige) Gestalt und damit gegenüber "Splitt" ein günstiges strömungstechnisches Verhalten, sie sind unter Betriebsbedingungen hydrolysestabil und zudem sehr kostengünstig herzustellen.

**Tabelle I**

| Erfindungsgemäße Träger und Trägerkatalysatoren | | | | | | |
|---|---|---|---|---|---|---|
| Träger-Nr. | Enzymaufnahme (U/ml Kat) | RG (ml/ml·h) | Testzeit (h) | Restaktivität (%) | Produktivität t_{HFS}/l Kat | Katalysator-Restmasse (%) |
| I. 1 | 7824 | 7,3 | 2250 | 50 | 9,6 | 97 |
| | | | 5000 | 20 | | |
| I. 2 | 8598 | 6,9 | 2550 | 50 | 9,7 | 98 |
| | | | 5240 | 20 | | |
| I. 3 | 7220 | 6,3 | 2150 | 50 | 7,5 | 98 |
| | | | 4400 | 20 | | |
| I. 4 | 7268 | 6,3 | 2950 | 50 | 9,2 | 97 |
| | | | 5081 | 20 | | |
| I. 5 | 7774 | 6,7 | nur Kurzeittest | | | 100 |
| Vergleich* | 7223 | 6,3 | 1650 | 50 | 5,2 | 12,5 |
| | | | 2758** | 29 | | |

| | | | | | | |
|---|---|---|---|---|---|---|
| * poröser SiO₂-Träger des Standes der Technik | | | | | | |
| ** Reaktor verstopft wegen Trägerauflösung | | | | | | |
| Kat = Katalysator RG = Raumgeschwindigkeit | | | | | | |

**Tabelle II**

| Nicht erfindungsgemäßes Vergleichsmaterial | | |
|---|---|---|
| Träger-Nr. | Enzymaufnahme (U/ml Kat) | Raumgeschwindigkeit (RG) (ml/ml·h) |
| II. V1 | 2850 | 2,3 |
| II. V2 a | 3205 | 2,8 |
| II. V2 b | 3310 | 2,9 |
| II. V2 c | 3250 | 2,8 |
| II. V3 | 3740 | 3,4 |
| Kat = Katalysator | | |

## Patentansprüche

1. Verfahren zur Herstellung von Enzymträgern auf anorganischer Basis und mit organischen Enzymbindungsstellen, bestehend aus einem rundlichen, makropermeablen Agglomerat von Primärpartikeln, die zu einer maulbeerartigen Substruktur angeordnet sind, dadurch gekennzeichnet, daß
a) man kugelförmige Primärpartikel aus mikroporösem α-Aluminiumoxidhydroxid der Formel AlO(OH) unter Verwendung eines organischen, vernetzungsfähige reaktive funktionelle Gruppen aufweisenden Binders so zu einer maulbeerartigen Substruktur aus zusammengelagerten Primärpartikeln granuliert, daß die Individualität der Primärpartikel darin im wesentlichen erhalten bleibt,
b) man das in a) erhaltene Agglomerat in seiner Form und Substruktur verfestigt, indem man den das Agglomerat durchsetzenden Binder mit einem, mit den reaktiven funktionellen Gruppen des Binders reagierenden organischen Härter zu einem dreidimensionalen, die maulbeerartige Substruktur durchdringenden Netzwerk aus organischem Polykondensat so umsetzt, daß
c) ein Teil der reaktiven Gruppen des organischen Binders und/oder des organischen Härters im Netzwerk bildenden Polykondensat als freie funktionelle Gruppen zur Enzymbindung erhalten bleibt.

2. Verfahren nach Anspruch 1, dadurch gekennzeichnet, daß als organische Binder Polyimine, vorzugsweise Polyethylenimin, eingesetzt werden.

3. Verfahren nach einem der vorhergehenden Ansprüche, dadurch gekennzeichnet, daß als organischer Härter Dialdehyde oder Epoxidverbindungen eingesetzt werden.

4. Verfahren nach Anspruch 3, dadurch gekennzeichnet, daß als Dialdehyd Glutardialdehyd verwendet wird.

5. Verfahren nach Anspruch 3, dadurch gekennzeichnet, daß als Epoxidverbindungen epoxyfunktionelle Siliconharze oder aliphatische Di- und Triepoxide verwendet werden.

6. Verfahren nach Anspruch 2, dadurch gekennzeichnet, daß man eine wäßrige Lösung des organischen Binders im Granulationsschritt unter a) einsetzt.

7. Verfahren nach Anspruch 6, dadurch gekennzeichnet, daß eine 5 bis 15 Gew.-%ige wäßrige Lösung des organischen Binders eingesetzt wird.

8. Verfahren nach Anspruch 7, dadurch gekennzeichnet, daß die wäßrige Polyiminlösung in einer Menge von etwa 0,5 kg pro 1 kg der Primärpartikel aus α-Aluminiumoxid-hydroxid eingesetzt wird.

9. Verfahren nach einem der Ansprüche 6 bis 8, dadurch gekennzeichnet, daß man die Primärpartikel unter Besprühen mit der wäßrigen Lösung des organischen Binders über einen Zeitraum von 5 bis 20 Minuten granuliert.

10. Verfahren nach einem der Ansprüche 6 bis 9, dadurch gekennzeichnet, daß man das in Granulationsschritt a) erhaltene Agglomerat unter weiterem Besprühen mit einer wäßrigen Lösung des organischen Binders über einen kurzen Zeitraum, vorzugsweise etwa 3 bis 5 Minuten, nachrollt.

11. Verfahren nach einem der vorhergehenden Ansprüche, dadurch gekennzeichnet, daß man im Granulationsschritt unter a) Agglomerate aus α-Aluminiumoxidhydroxid herstellt, die einen Gehalt an organischen Binder von 3 bis 7 Gew.-%, vorzugsweise von 4 bis 6 Gew.-%, aufweisen.

12. Enzymträger auf anorganischer Basis und mit organischen funktionellen Enzymbindungsstellen, bestehend aus einem rundlichen makropermeablen Agglomerat von Primärpartikeln, die zu einer maulbeerartigen Substruktur angeordnet sind, dadurch gekennzeichnet, daß
a) die maulbeerartige Substruktur aus zusammengelagerten, kugelförmigen Primärpartikeln aus mikroporösem α-Aluminiumoxidhydroxid der Formel AlO(OH) gebildet ist und die Individualität der Primärpartikel darin im wesentlichen erhalten ist,
b) das Agglomerat in seiner Form und Struktur durch ein dreidimensionales, die maulbeerartige Substruktur durchdringendes Netzwerk, bestehend aus einem organischen Polykondensat, verfestigt ist, und
c) das Netzwerk bildende organische Polykondensat noch freie organische funktionelle Gruppen als Bindungsstellen für Enzyme besitzt.

13. Trägargebundene Enzyme (Trägerkatalysatoren), dadurch gekennzeichnet, daß Enzyme, vorzugsweise Glucoseisomerase, an einen Träger gemäß Anspruch 12 immobilisiert sind.

## Claims

1. Process for preparing inorganic-based enzyme substrates having organic enzyme binding sites and consisting of a round, macropermeable agglomerate of primary particles arranged to form a mulberry-type substructure, characterised in that
a) spherical primary particles of microporous α-aluminium oxide hydroxide of the formula AlO(OH) are granulated using an organic binder having cross-linkable reactive functional groups to form a mulberry-type substructure of aggregated primary particles, so that the individuality of the primary particles is essentially retained therein,
b) the shape and substructure of the agglomerate obtained in a) is strengthened by reacting the binder interspersing the agglomerate with an organic hardener reacting with the reactive functional groups of the binder to form a three-dimensional network of organic polycondensate penetrating the mulberry-type substructure, so that
c) some of the reactive groups of the organic binder and/or organic hardener are retained as free functional groups for enzyme bonding in the network-forming polycondensate.

2. Process according to claim 1, characterised in that polyimines, preferably polyethyleneimine, are used as organic binder.

3. Process according to one of the preceding claims, characterised in that dialdehydes or epoxy compounds are used as organic hardeners.

4. Process according to claim 3, characterised in that glutardialdehyde is used as dialdehyde.

5. Process according to claim 3, characterised in that epoxy-functional silicon resins or aliphatic diepoxides and triepoxides are used as epoxy compounds.

6. Process according to claim 2, characterised in that an aqueous solution of the organic binder is used in the granulation step under a).

7. Process according to claim 6, characterised in that a 5 to 15 wt.% strength aqueous solution of the organic binder is used.

8. Process according to claim 7, characterised in that the aqueous polyimine solution is used in an amount of approximately 0.5 kg per 1 kg of the primary particles of α-aluminium oxide hydroxide.

9. Process according to one of claims 6 to 8, characterised in that the primary particles are granulated over a period of 5 to 20 minutes while being sprayed with the aqueous solution of the organic binder.

10. Process according to one of claims 6 to 9, characterised in that the agglomerate obtained in the granulation step a) is rolled over a short period, preferably approximately 3 to 5 minutes, while continuing spraying with an aqueous solution of the organic binder.

11. Process according to one of the preceding claims, characterised in that agglomerates of α-aluminium oxide hydroxide, which contain 3 to 7 wt.%, preferably 4 to 6 wt.%, of organic binder, are prepared in the granulation step under a).

12. Inorganic-based enzyme substrate having organic functional enzyme binding sites and consisting of a round, macropermeable agglomerate of primary particles which are arranged to form a mulberry-type substructure, characterised in that
a) the mulberry-type substructure is formed from aggregated, spherical primary particles of microporous α-aluminium oxide hydroxide of the formula AlO(OH) and the individuality of the primary particles is essentially retained therein,
b) the shape and structure of the agglomerate is strengthened by a three-dimensional network penetrating the mulberry-type substructure and consisting of an organic polycondensate, and
c) the network-forming organic polycondensate still has free organic functional groups as binding sites for enzymes.

13. Substrate-bound enzymes (substrate catalysts), characterised in that enzymes, preferably glucose isomerase, are immobilised on a substrate according to claim 12.

## Revendications

1. Procédé de préparation de supports d'enzymes à base minérale et comportant des sites organiques de liaison d'enzymes, consistant en un agglomérat macroperméable arrondi de particules primaires, qui sont agencées en une sous-structure ressemblant à une mûre, procédé caractérisé en ce que :
a) on soumet des particules primaires sphériques en d'α oxyde-hydroxyde d'aluminium microporeux de formule AlO(OH) à granulation à partir des particules primaires réunies avec utilisation d'un liant organique, présentant des groupes fonctionnels réactifs capables de provoquer une réticulation pour obtenir une sous-structure ressemblant à une mûre, de manière que l'individualité des particules primaires demeure essentiellement conservée,
b) on consolide dans sa forme et sa sous-structure l'agglomérat obtenu en (a) en faisant réagir le liant, qui traverse l'agglomérat, avec un durcisseur organique pouvant réagir avec les groupes fonctionnels réactifs du liant en donnant un réseau tridimensionnel de polycondensat organique pénétrant la sous-structure ressemblant à une mûre, de façon que :
c) une partie des groupes réactifs du liant organique et/ou du durcisseur organique soit conservée, dans le polycondensat formant le réseau, sous forme de groupes fonctionnels libres pour la liaison des enzymes.

2. Procédé selon la revendication 1, caractérisé en ce qu'on utilise comme liant organique des polyimines, avantageusement de la polyéthylène-imine.

3. Procédé selon l'une des revendications précédentes, caractérisé en ce qu'on utilise comme durcisseur organique des dialdéhydes ou des époxydes.

4. Procédé selon la revendication 3, caractérisé en ce qu'on utilise comme dialdéhyde le glutarodialdéhyde.

5. Procédé selon la revendication 3, caractérisé en ce qu'on utilise comme époxydes des résines de silicone comportant des groupes époxydes fonctionnels ou des diépoxydes et des triépoxydes aliphatiques.

6. Procédé selon la revendication 2, caractérisé en ce qu'on utilise une solution aqueuse du liant organique à l'étape de granulation effectuée en (a).

7. Procédé selon la revendication 6, caractérisé en ce qu'on utilise une solution aqueuse contenant 5 à 15 % en poids du liant organique.

8. Procédé selon la revendication 7, caractérisé en ce qu'on utilise la solution aqueuse de polyimine en une quantité d'environ 0,5 kg pour 1 kg des particules primaires en α-oxyde-hydroxyde d'aluminium.

9. Procédé selon l'une des revendications 6 à 8, caractérisé en ce qu'on granule les particules primaires en les aspergeant de la solution aqueuse du liant organique, en un espace de temps de 5 à 20 minutes.

10. Procédé selon l'une des revendications 6 à 9, caractérisé en ce que on soumet l'agglomérat obtenu dans l'étape de granulation (a), en continuant de l'asperger avec une solution aqueuse du liant organique, à un post-roulage pendant un court espace de temps, avantageusement environ 3 à 5 minutes.

11. Procédé selon l'une des revendications précédentes, caractérisé en ce qu'on prépare, dans l'étape de granulation en (a), des agglomérats en d'α-oxyde-hydroxyde d'aluminium qui présentent une teneur en liant organique de 3 à 7 % en poids, avantageusement de 4 à 6 % en poids.

12. Support d'enzyme(s) à base minérale et comportant des sites fonctionnels organiques pour la liaison d'enzymes, ce support consistant en un agglomérat macroperméable arrondi de particules primaires, qui sont agencées en une sous-structure ressemblant à une mûre, support caractérisé en ce que :
a) la sous-structure ressemblant à une mûre est formée par des particules primaires sphériques regroupées, en α-oxyde-hydroxyde d'aluminium microporeux de formule AlO(OH), et en ce que l'individualité des particules primaires y est essentiellement maintenue,
b) l'agglomérat est consolidé en sa forme et sa structure grâce à un réseau tridimensionnel, pénétrant la sous-structure ressemblant à une mûre et consistant en un polycondensat organique, et
c) le polycondensat organique formateur du réseau possède encore des groupes fonctionnels organiques libres pouvant jouer le rôle de site(s) de liaison ou de fixation d'enzymes.

13. Enzymes fixées ou immobilisées sur un support (catalyseurs sur support), caractérisé(e)(s) en ce que les enzymes, avantageusement de la glucose isomérase, sont immobilisées sur un support selon la revendication 12.
